# EUROPÄISCHE PATENTSCHRIFT

(11) **EP 1 301 509 B1**
(45) Veröffentlichungstag und Bekanntmachung des Hinweises auf die Patenterteilung: **06.10.2004**
(21) Anmeldenummer: 01984160.0
(22) Anmeldetag: 12.06.2001
(51) Int. Cl.: C07D 413/10, C07D 413/12

(54) **MULTIFUNKTIONELLE KOPPLUNGSAGENZIEN MIT ZYKLISCHEN IMINOESTER- UND IMINOETHERGRUPPEN, VERFAHREN ZU DEREN HERSTELLUNG UND VERWENDUNG**
MULTIFUNCTIONAL COUPLING AGENTS WITH CYCLIC IMINOESTER AND IMINOETHER GROUPS, METHOD FOR PRODUCING THE SAME AND USE THEREOF
AGENTS DE COUPLAGE MULTIFONCTIONNELS A GROUPES IMINOESTER ET IMINOETHER CYCLIQUES, LEUR PROCEDE DE PRODUCTION ET LEUR UTILISATION

(30) Priorität: 07.07.2000 DE 10034154
(43) Veröffentlichungstag der Anmeldung: 16.04.2003
(73) Patentinhaber: Institut für Polymerforschung Dresden E.V., 01069 Dresden (DE)
(72) Erfinder: BÖHME, Frank, 01705 Pesterwitz (DE); JACKISCH, Lothar, 08056 Zwickau (DE)
(74) Vertreter: Rauschenbach, Marion, Dipl.-Ing.
(86) Internationale Anmeldenummer: PCT/DE2001/002257
(87) Internationale Veröffentlichungsnummer: WO 2002/004441

(56) Entgegenhaltungen:
- DE-C- 19 812 409
- US-A- 3 408 326

## Beschreibung

### Anwendungsgebiete der Erfindung

Die Erfindung bezieht sich auf das Gebiet der Chemie, insbesondere der Polymerchemie und betrifft multifunktionelle Kopplungsagenzien mit zyklischen Iminoester- und Iminoethergruppen, die beispielsweise zur Synthese von funktionalisierten Blockcopolymeren und in Polymerblends eingesetzt werden und ein Verfahren zu deren Herstellung und Verwendung.

### Stand der Technik

Bis-2-oxazoline und die strukturell ähnlichen Bis-2-oxazine werden als bifunktionelle Kopplungsagenzien insbesondere für carboxylgruppenhaltige Polymere in der Literatur und in zahlreichen Patentschriften beschrieben.
Als zyklische Iminoether können derartige Verbindungen über eine ringöffnende Additionsreaktion mit Carboxylgruppen zu entsprechenden Esteramidstrukturen reagieren. Darüber hinaus sind Additionsreaktionen mit weiteren protonenhaltigen Nukleophilen wie Phenole und Thiole bekannt.

Hieraus ergeben sich vielfältige Möglichkeiten zum Aufbau und zur Modifizierung von Polymeren. So kann beispielsweise das Molekulargewicht von carboxylgruppenterminierten Polyestern durch den Einsatz von Bis-2-oxazolinen als Kettenverlängerer erhöht werden (H. Inata, S. Matsumura, J. Appl. Polym. Sci. 30, 3325 (1985); T. Loontjens, W. Belt, D. Stanssens, P. Weerts, Pol. Bull. 30, 13 (1993), Y. Sano, J. Pol. Sci., Polym. Chem. Ed., 27, 2749 (1989); F. Böhme, D. Leistner, A. Baier, Angew. Makromol. Chem., 224, 167 (1995)). Hierzu existiert eine Vielzahl an Patentschriften, die den Einsatz von Bis-2-oxazolinen als Kettenverlängerer und Stabilisatoren bei der reaktiven Verarbeitung von Polyestern beschreiben.

Bekannt sind weiterhin Polyadditionsreaktionen von Bis-2-oxazolinen mit Di- oder Polycarbonsäuren, -phenolen und -thiolen zum Aufbau von Polymeren sowie die Synthese von Blockcopolymeren durch Reaktion von carboxylgruppenterminierten Oligomeren mit Bis-2-oxazolinen und Bis-2-oxazinen.

Bis-2-oxazinone bzw. Bis-2-oxazolone stellen als zyklische Iminoester ebenfalls bifunktionelle Kopplungsagenzien dar, die mit Aminogruppen und aliphatischen Hydroxylgruppen in einer ringöffnenden Additionsreaktion reagieren können. So werden entsprechende Verbindungen beispielsweise als effektive Kettenverlängerer in Polyethylenterephthalat und Polybutylenterephthalat, welche aliphatische Hydroxylgruppen enthalten, beschrieben (H. Inata, S. Matsumura, J. Appl. Polym. Sci., 32, 4581 (1986) und 34, 2609 (1987)). Weiterhin ist bekannt, daß Bis-2-oxazinone bzw. Bis-2-oxazolone im Gemisch mit Bis-2-oxazolinen bzw. Bis-2-oxazinen als Kettenverlängerer in Polyesterschmelzen eingesetzt werden können, wodurch durch Reaktion sowohl der Hydroxyl- als auch der Carbonsäureendgruppen ein effektiver Molmassenaufbau möglich ist (JP 59053529).

Des weiteren ist bekannt, daß zyklische Iminoester in Schmelze oder Lösung mit Diaminen oder aminoterminierten Oligomeren zu entsprechenden Copolymeren reagieren können (US 3,408,326; M. Acevedo, A. Fradet, J. Polym. Sci., Polym. Chem., 31, 817 (1993) und 31, 1579, (1993)). Ebenfalls bekannt ist die Reaktion mit hydroxyterminierten Oligomeren (M. Acevedo, A. Fradet, Polym. Prepr., Am. Chem. Soc., Div. Polym. Chem., 34, 457 (1993)).

Darüber hinaus sind weitere bifunktionelle Kopplungsagenzien, die über eine ringöffnende Additionsreaktion mit unterschiedlichen funktionellen Gruppen reagieren können, beschrieben. An dieser Stelle seien als Beispiele Bislactamate (T. Loontjens, K. Pauwels, F. Derks, M. Neilen, C. K. Sham, M. Seme, J. Appl. Polym. Sci., 65, 1813 (1997)) und Bisimidazoline (T. Fischer, H. Levebvre, A. Fradet; Macromol. Symp., 118, 79 (1997); ), Diepoxide und zyklische Dianhydride genannt.

Weiterhin ist eine Vielzahl an di- und polyfunktionellen Verbindungen, beispielsweise Diisocyanate, bekannt, die als Kopplungsagenzien in unterschiedlichen Polymersystemen eingesetzt werden.

Im Vergleich zu den weiter oben beschriebenen di- oder polyfunktionellen Kopplungsagenzien, die jeweils nur einen Typ einer funktionellen Gruppe enthalten, besitzen Kopplungsagenzien, die über Additionsreaktionen reagieren und verschiedene funktionelle Gruppen unterschiedlicher Reaktivität im Molekül enthalten (L. Jakisch, H. Komber, L. Häußler, F. Böhme, Macromol. Symp., 149, 237 (2000)), den Vorteil der selektiven Kopplung von chemischen Verbindungen und Polymeren mit unterschiedlichen funktionellen Gruppen.
Die in DE 198 12 409 C1 angegebenen Kopplungsagenzien enthalten zyklische Iminoester- und Iminoethergruppen, wobei die Iminoestergruppen mit Aminogruppen und aliphatischen Hydroxylgruppen und die Iminoethergruppen mit Carboxylgruppen selektiv in Lösung und in Schmelze reagieren, wodurch z.B. bei der in-situ Kompatibilisierung von Polymerblends mittels bifunktioneller Kopplungsagenzien ausschließlich Kopplungsreaktionen zwischen den beiden Blendkomponenten in der Phasengrenzfläche realisierbar sind und bei der Synthese von Block- und Multiblockcopolymeren ein definierter Polymeraufbau ermöglicht wird.

Die Herstellung der in DE 198 12 409 C1 angegebenen Kopplungsagenzien erfolgt auf Basis von di- oder trifunktionellen Carbonsäureesterchloriden.

Kopplungsagenzien mit zyklischen Iminoester- und Iminoethergruppen, die darüber hinaus zusätzliche, aktivierbare funktionelle Gruppen enthalten, die für nachträgliche chemische Reaktionen oder physikalische Wechselwirkungen zur Verfügung stehen, sind bisher nicht bekannt.

### Beschreibung der Erfindung

Der Erfindung liegt die Aufgabe zugrunde, Kopplungsagenzien mit zwei funktionellen Gruppen unterschiedlicher Reaktivität anzugeben, die selektiv über Additionsreaktionen mit zwei weiteren, unterschiedlichen reaktiven Gruppen reagieren und selbst nicht miteinander reagieren sowie eine oder mehrere zusätzliche, aktivierbare funktionelle Gruppen enthalten, die für chemische Reaktionen oder physikalische Wechselwirkungen zur Verfügung stehen.

Erfindungsgemäße Verbindungen sind multifunktionelle Kopplungsagenzien mit zyklischen Iminoester- und Iminoethergruppen der allgemeinen Struktur nach Formel 1, 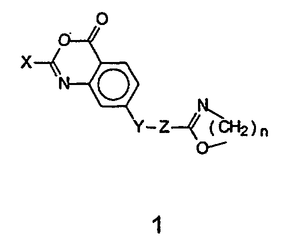 wobei X ein Aryl-, Aralkyl-, Aralkylen-, Alkyl- oder Alkylenrest mit 1 bis 42 Kohlenstoffatomen und 0 bis 12 Heteroatomen ist und eine oder mehrere zusätzliche, aktivierbare funktionelle Gruppen enthält, die für chemische Reaktionen oder physikalische Wechselwirkungen zur Verfügung stehen, Y eine -C(O)NH- oder -C(O)O-Gruppe oder eine chemische Bindung darstellt, Z ein Aryl-, Aralkyl- oder Alkylrest oder eine chemische Bindung und n gleich 2 oder 3 ist.

In einer vorteilhaften Ausführungsform stellt die Gruppierung Y-Z entsprechend der allgemeinen Struktur nach Formel 1 eine chemische Bindung dar, woraus sich eine allgemeine Struktur nach Formel 2 ergibt.

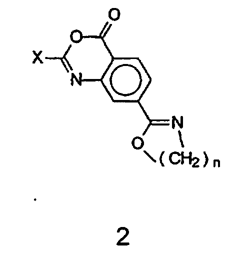

Weiterhin vorteilhaft ist es, wenn entsprechend der allgemeinen Struktur nach Formel 1 Y eine -C(O)NH- Gruppe und Z ein 1,4-Phenylenrest und n gleich 2 ist, woraus sich folgende allgemeine Struktur nach Formel 3 ergibt.

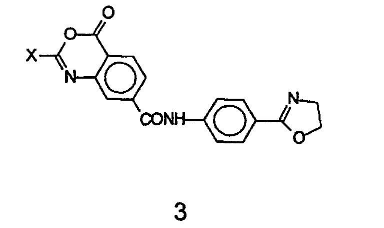

Besonders vorteilhafte Ausführungsformen stellen Verbindungen entsprechend der allgemeinen Strukturen nach den Formeln 1 - 3 dar, in denen die zusätzlichen, aktivierbaren funktionellen Gruppen von X eine chemisch oder physikalisch vernetzbare Gruppe, eine polymerisierbare Gruppe, eine chromophore Gruppe, eine gegen Oxidation oder Radikalbildung stabilisierende Gruppe, eine mit spektroskopischen Methoden nachweisbare Gruppe, die als Marker dient, oder eine gegenüber Metallionen komplexierende Gruppe sind.

Die erfindungsgemäßen Verbindungen entsprechend Anspruch 1 werden erfindungsgemäß mittels an sich bekannter Verfahrensschritte, die in neuer Art und Weise miteinander kombiniert werden, hergestellt.

Dabei wird von Carbonsäurechloriden der allgemeinen Struktur nach Formel 4 ausgegangen, wobei X ein Aryl-, Aralkyl-, Aralkylen-, Alkyl- oder Alkylenrest mit 1 bis 42 Kohlenstoffatomen und 0 bis 12 Heteroatomen ist und eine oder mehrere zusätzliche, aktivierbare funktionelle Gruppen enthält, die für chemische Reaktionen oder physikalische Wechselwirkungen zur Verfügung stehen.

X-COCl 4

Im Gegensatz zu den nach dem Stand der Technik aus DE 198 12 409 C1 bekannten Aminosäuren, wie z.B. Anthranilsäure wird Aminoterephthalsäure als weiterer Synthesebaustein verwendet. Hieraus ergeben sich wesentliche Vorteile beim Einbau der 2-Oxazolin- und 2-Oxazinongruppen. So entfallen die in DE 198 12 409 C1 beschriebenen partiellen Verseifungen der Estergruppen der als Ausgangsstoff verwendeten Carbonsäurederivate. Zugleich ist es durch die nachfolgend beschriebene neuartige Synthese möglich, durch den Einsatz von Carbonsäurechloriden, die zusätzliche, aktivierbare funktionelle Gruppen enthalten, letztere in einfacher Art und Weise in das Kopplermolekül einzubauen.

Die Synthese der erfindungsgemäßen Verbindungen erfolgt durch Umsetzung von Verbindungen der allgemeinen Struktur nach Formel 4 mit Aminoterephthalsäure zu Verbindungen der allgemeinen Struktur nach Formel 5 in einem inerten

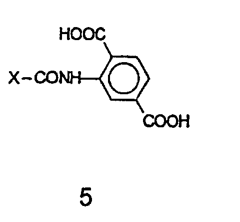

Lösungsmittel in Gegenwart eines Säurefängers, vorzugsweise einem tertiären Amin, bei Temperaturen von 0 °C bis 150 °C, vorzugsweise von 0 °C bis 20 °C, wobei X ein Aryl-, Aralkyl-, Aralkylen-, Alkyl- oder Alkylenrest mit 1 bis 42 Kohlenstoffatomen und 0 bis 12 Heteroatomen ist und eine oder mehrere zusätzliche, aktivierbare funktionelle Gruppen enthält, die für chemische Reaktionen oder physikalische Wechselwirkungen zur Verfügung stehen (identisch mit X in Formel 4).

Als inerte Lösungsmittel können aromatische Kohlenwasserstoffe, Halogenalkane oder dipolar, aprotische Lösungsmittel wie z. B. Dimethylacetamid Verwendung finden. Vorzugsweise werden jedoch Ether, speziell Tetrahydrofuran verwendet.
Nach der Isolierung der Zwischenprodukte der allgemeinen Struktur nach Formel 5 durch z.B. Abdestillieren des Lösungsmittels und Auswaschen des entstandenen Triethylammoniumchlorides mit Wasser werden diese nach der Trocknung durch Umsetzung mit zugleich wasserentziehenden Chlorierungsagenzien bei Temperaturen 0 °C bis 150 °C, vorzugsweise in siedendem Thionylchlorid, in die Verbindungen entsprechend der allgemeinen Struktur nach Formel 6 überführt, 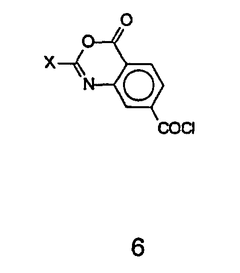 wobei X wiederum identisch mit denen in Formel 4 ist, und nachfolgend mit 2-Aminoethanol oder 3-Aminopropanol in einem inerten Lösungsmittel unter Zusatz eines Säurefängers, vorzugsweise einem tertiären Amin, bei Temperaturen von 0 °C bis 150 °C, vorzugsweise bei 0°C bis 20°C umgesetzt.
Als inerte Lösungsmittel können aromatische Kohlenwasserstoffe, Halogenalkane oder dipolar, aprotische Lösungsmittel wie z.B. Dimethylacetamid Verwendung finden.
Vorzugsweise werden jedoch Ether, speziell Tetrahydrofuran verwendet.
Anschließend erfolgt die Reaktion mit Thionylchlorid bei Temperaturen von 0 °C bis 20 °C in Gegenwart eines inerten Lösungsmittels, vorzugsweise Dichlormethan. Danach wird mit einer Base, vorzugsweise wäßrige Natriumbicarbonatlösung oder ein tertiäres Amin, behandelt. Es entsteht eine erfindungsgemäße Verbindung.

In einer zweiten Variante werden die Verbindungen entsprechend der allgemeinen Struktur nach Formel 6 mit einem in 2-Stellung substituierten Oxazolin oder Oxazin, welches im Substituenten eine Hydroxylgruppe oder eine primäre Aminogruppe enthält, vorzugsweise mit 2(4-Aminophenyl)-1,3-oxazolin, in einem inerten Lösungsmittel unter Zusatz eines Säurefängers, vorzugsweise einem tertiären Amin, bei Temperaturen von 0 °C bis 150 °C, vorzugsweise bei 0°C bis 20°C, umgesetzt.
Als inerte Lösungsmittel können wiederum aromatische Kohlenwasserstoffe, Halogenalkane oder dipolar, aprotische Lösungsmittel wie z.B. Dimethylacetamid Verwendung finden. Vorzugsweise werden jedoch Ether, speziell Tetrahydrofuran verwendet. Es entsteht eine erfinduhgsgemäße Verbindung.

Die erfindungsgemäßen multifunktionellen Kopplungsagenzien mit zyklischen lminoester- und Iminoethergruppen zeichnen sich dadurch aus, daß ihre zyklischen Iminoester- und Iminoethergruppen in Gegenwart nukleophiler Gruppen wie aliphatische und aromatische Carboxyl- und Aminogruppen sowie aliphatische und phenolische Hydroxylgruppen deutlich unterschiedliche Reaktivitäten aufweisen, wodurch eine selektive Kopplung ermöglicht wird und die im Kopplermolekül enthaltenen zusätzlichen, aktivierbaren funktionellen Gruppen für chemische Reaktionen oder physikalische Wechselwirkungen zur Verfügung stehen.

Überraschenderweise erfolgen die Kopplungsreaktionen bezüglich Effektivität und Selektivität in ähnlicher Art und Weise wie bei den Verbindungen, die in der DE 198 12 409 C1 beschrieben sind, obwohl die zyklischen Iminoether- und Iminoestergruppen strukturell anders angeordnet sind.

Die erfindungsgemäßen Verbindungen können zur Kettenverlängerung, Verzweigung oder Vernetzung, zur Bildung von Blockcopolymeren, Multiblockcopolymeren und zur chemischen Kopplung in reaktiven Polymerblends und Composites verwendet werden, wobei jeweils eine Komponente Carboxylgruppen und/oder phenolische Gruppen und die andere Komponente Amino- und/oder aliphatische Hydroxylgruppen enthält. Zu dem Vorteil, daß die Kopplungsreaktionen in derartigen Polymerblends und Composites ausschließlich in der Grenzschicht beider Komponenten stattfinden und die innere Struktur der einzelnen Komponenten (Molekulargewicht, chemische Struktur) nicht beeinflußt wird, wird zugleich mit der Lokalisierung einer oder mehrerer zusätzlicher, aktivierbarer funktioneller Gruppen, die für chemische Reaktionen oder physikalische Wechselwirkungen zur Verfügung stehen, in der Grenzschicht eine Modifizierung oder Markierung dieser ermöglicht.

Der Einsatz der erfindungsgemäßen Verbindungen bei der Synthese von Block- und Multiblockcopolymeren ermöglicht die Herstellung von entsprechend funktionalisierten Polymeren, die damit ebenfalls einer Modifizierung zugänglich sind.

### Bester Weg zur Ausführung der Erfindung

Im weiteren wird die Erfindung an den folgenden Ausführungsbeispielen näher erläutert.

### Beispiel 1

### Synthese und Anwendung von 7-(1,3-Oxazolin-2-yl)-2-phenyl-benzo[d][1,3]oxazin-4-one (Formel 7)

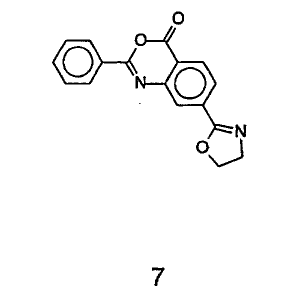

Zu einer Lösung von 14,05 g Benzoylchlorid in 250 ml wasserfreiem Tetrahydrofuran werden bei 0°C bis 10°C 18,10 g 2-Aminobenzoesäure zugegeben und anschließend bei dieser Temperatur 10,10 g Triethylamin innerhalb von 30 min zugetropft. Danach wird noch eine Stunde bei Raumtemperatur gerührt und anschließend das Lösungsmittel im Vakuum abdestilliert. Der feste Rückstand wird mit 200 ml Wasser aufgenommen, abgesaugt und mit Wasser gewaschen. Das erhaltene Produkt wird in 150 ml Thionylchlorid 4 Stunden unter Rückfluß erhitzt. Anschließend wird das überschüssige Thionylchlorid vollständig im Vakuum abdestilliert und danach der Rückstand in 300 ml wasserfreiem Tetrahydrofuran gelöst und bei 0°C bis 10°C mit einer Mischung aus 6,10 g 2-Ethanolamin und 10,10 g Triethylamin innerhalb von 30 min versetzt. Anschließend wird eine Stunde bei Raumtemperatur gerührt, der entstandene Niederschlag abgesaugt und mit Tetrahydrofuran und Wasser gewaschen. Nach dem Trocknen im Vakuum wird das Produkt in eine Lösung von 23,8 g Thionylchlorid in 300 ml wasserfreiem Methylenchlorid bei -5°C bis 0°C suspendiert und 6 Stunden bei dieser Temperatur gerührt. Danach wird der Niederschlag abgesaugt, mit Methylenchlorid gewaschen und im Vakuum getrocknet. Anschließend wird das erhaltene Produkt in 300 ml einer wäßrigen Lösung von 8,40 g Natriumbicarbonat suspendiert, 10 min gerührt, der Niederschlag abgesaugt und mit Wasser gewaschen.

Danach wird das Produkt mit 200 ml Aceton 10 Minuten unter Rückfluß erhitzt und anschließend abgesaugt und im Vakuum getrocknet.
Man erhält 16,75 g 7-(1,3-Oxazolin-2-yl)-2-phenyl-benzo[*d*][1,3]oxazin-4-one mit einem Schmelzpunkt von 221 °C nach Umkristallisation in Ethanol.
Die Struktur wurde mittels ¹H- und ¹³C-NMR-Spektroskopie überprüft.
Die Substanz reagiert mit äquimolaren Mengen Dodecylamin in siedendem Toluol oder in Schmelze in einem Temperaturbereich von 100 - 220 °C unter Öffnung des Oxazinonrings und Erhalt der Oxazolingruppe.
Die Substanz reagiert mit äquimolaren Mengen Laurinsäure in Schmelze in einem Temperaturbereich von 150 - 280 °C unter Öffnung des Oxazolinrings und Erhalt der Oxazinongruppe.
Die Zugabe von 7-(1,3-Oxazolin-2-yl)-2-phenyl-benzo[*d*][1,3]oxazin-4-one in eine Schmelze von Polyamid 6, welches Amino- und Carbonsäuregruppen enthält, bewirkt eine Erhöhung der Schmelzeviskosität durch Kopplung der Endgruppen und daraus resultierenden höheren Molmassen.

### Beispiel 2

### Synthese und Anwendung von 4-Oxo-2-phenyl-4H-benzo[d][1,3]oxazine-7-carboxylic acid [4-(1,3-oxazolin-2-yl)-phenyl]-amide (Formel 8)

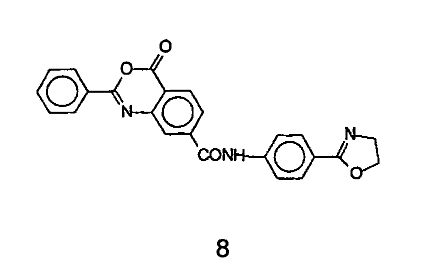

Zu einer Lösung von 14,05 g Benzoylchlorid in 250 ml wasserfreiem Tetrahydrofuran werden bei 0°C bis 10°C 18,10 g 2-Aminobenzoesäure zugegeben und anschließend bei dieser Temperatur 10,10 g Triethylamin innerhalb von 30 min zugetropft. Danach wird noch eine Stunde bei Raumtemperatur gerührt und anschließend das Lösungsmittel im Vakuum abdestilliert. Der feste Rückstand wird mit 200 ml Wasser aufgenommen, abgesaugt und mit Wasser gewaschen. Das erhaltene Produkt wird in 150 ml Thionylchlorid 4 Stunden unter Rückfluß erhitzt. Anschließend wird das überschüssige Thionylchlorid vollständig im Vakuum abdestilliert und danach der Rückstand in 300 ml wasserfreiem Tetrahydrofuran gelöst und bei 0°C bis 10°C mit einer Mischung aus 16,20 g 2(4-Aminophenyl)-oxazolin und 10,10 g Triethylamin in 100 ml wasserfreiem Tetrahydrofuran innerhalb von 30 min versetzt. Anschließend wird eine Stunde bei Raumtemperatur gerührt, der entstandene Niederschlag abgesaugt und mit Tetrahydrofuran und Wasser gewaschen. Danach wird das Produkt mit 200 ml Ethanol 10 Minuten unter Rückfluß erhitzt und anschließend abgesaugt und im Vakuum getrocknet.
Man erhält 34,92 g 4-Oxo-2-phenyl-4*H*-benzo[*d*][1,3]oxazine-7-carboxylic acid [4-(1,3-oxazolin-2-yl)-phenyl]-amide.
Die Substanz besitzt keinen Schmelzpunkt.
Die Stuktur wurde mittels ¹H- und ¹³C-NMR-Spektroskopie überprüft.
Die Zugabe von 4-Oxo-2-phenyl-4*H*-benzo[*d*][1,3]oxazine-7-carboxylic acid [4-(1,3-oxazolin-2-yl)-phenyl]-amide in eine Schmelze von Polyamid 6, welches Aminound Carbonsäuregruppen enthält, bewirkt eine Erhöhung der Schmelzeviskosität durch Kopplung der Endgruppen und daraus resultierenden höheren Molmassen.

### Beispiel 3

### Synthese und Anwendung von 2-(4-Allyloxy-phenyl)-4-oxo-4H-benzo[d][1,3]oxazine-7-carboxylic acid [4-(1,3-oxazolin-2-yl)-phenyl]-amide (Formel 9)

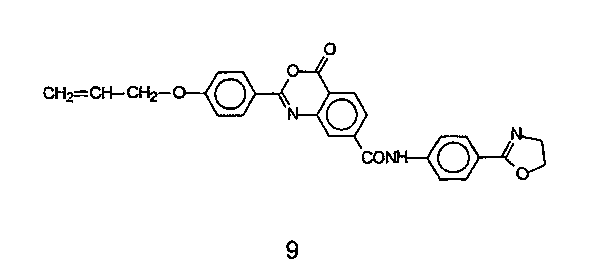

Zu einer Lösung von 19,65 g 4-Allyloxybenzoylchlorid in 250 ml wasserfreiem Tetrahydrofuran werden bei 0°C bis 10°C 18,10 g 2-Aminobenzoesäure zugegeben und anschließend bei dieser Temperatur 10,10 g Triethylamin innerhalb von 30 min zugetropft. Danach wird noch eine Stunde bei Raumtemperatur gerührt und anschließend das Lösungsmittel im Vakuum abdestilliert. Der feste Rückstand wird mit 200 ml Wasser aufgenommen, abgesaugt und mit Wasser gewaschen. Das erhaltene Produkt wird in 150 ml Thionylchlorid 6 Stunden bei einer Temperatur von 60 °C gerührt. Anschließend wird das überschüssige Thionylchlorid vollständig im Vakuum abdestilliert und danach der Rückstand in 300 ml wasserfreiem Tetrahydrofuran gelöst und bei 0°C bis 10°C mit einer Mischung aus 16,20 g 2(4-Aminophenyl)-oxazolin und 10,10 g Triethylamin in 100 ml wasserfreiem Tetrahydrofuran innerhalb von 30 min versetzt. Anschließend wird eine Stunde bei Raumtemperatur gerührt, der entstandene Niederschlag abgesaugt und mit Tetrahydrofuran und Wasser gewaschen. Danach wird das Produkt mit 200 ml Ethanol 10 Minuten unter Rückfluß erhitzt und anschließend abgesaugt und im Vakuum getrocknet. Man erhält 32,45 g 2-(4-Allyloxy-phenyl)-4-oxo-4*H*-benzo[*d*][1,3]oxazine-7-carboxylic acid [4-(1,3-oxazolin-2-yl)-phenyl]-amide.
Die Substanz besitzt keinen Schmelzpunkt.
Die Stuktur wurde mittels ¹H- und ¹³C-NMR-Spektroskopie überprüft.
Die Substanz reagiert mit äquimolaren Mengen Dodecylamin in siedendem Toluol oder in Schmelze in einem Temperaturbereich von 100 - 220 °C unter Öffnung des Oxazinonrings und Erhalt der Oxazolingruppe und der Allylgruppe.

Die Substanz reagiert mit äquimolaren Mengen Laurinsäure in Schmelze in einem Temperaturbereich von 150 - 220 °C unter Öffnung des Oxazolinrings und Erhalt der Oxazinongruppe und der Allylgruppe.
Die Substanz reagiert mit jeweils äquimolaren Mengen Dodecylamin und Laurinsäure im Gemisch in Schmelze in einem Temperaturbereich von 150 - 220 °C unter Öffnung des Oxazolin- und Oxazinonrings und unter Erhalt der Allylgruppe.

### Beispiel 4

### Synthese und Anwendung von 2-Anthracen-9-yl-4-oxo-4H-benzo[d][1,3]oxazine-7-carboxylic acid [4-(1,3-oxazolin-2-yl)-phenyl]-amide (Formel 10)

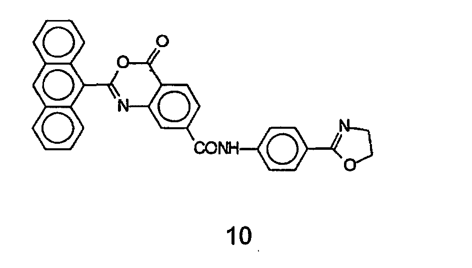

Zu einer Lösung von 2,525 g 9-Anthracencarbonsäurechlorid in 25 ml wasserfreiem Tetrahydrofuran werden bei 0°C bis 10°C 1,81 g 2-Aminobenzoesäure zugegeben und anschließend bei dieser Temperatur 1,01 g Triethylamin in 5 ml wasserfreiem Tetrahydrofuran zugetropft. Danach wird noch eine Stunde bei Raumtemperatur gerührt und anschließend das Lösungsmittel im Vakuum abdestilliert. Der feste Rückstand wird mit 50 ml Wasser aufgenommen, abgesaugt und mit Wasser gewaschen. Das erhaltene Produkt wird in 25 ml Thionylchlorid 6 Stunden unter Rückfluß erhitzt. Anschließend wird das überschüssige Thionylchlorid vollständig im Vakuum abdestilliert und danach der Rückstand in 100 ml wasserfreiem Tetrahydrofuran gelöst und bei 0°C bis 10°C mit einer Mischung aus 1,62 g 2(4-Aminophenyl)-oxazolin und 1,01 g Triethylamin in 20 ml wasserfreiem Tetrahydrofuran innerhalb von 10 min versetzt. Anschließend wird eine Stunde bei Raumtemperatur gerührt, der entstandene Niederschlag abgesaugt und mit Tetrahydrofuran und Wasser gewaschen. Danach wird das Produkt mit 100 ml Ethanol 10 Minuten unter Rückfluß erhitzt und anschließend abgesaugt und im Vakuum getrocknet.
Man erhält 3,73 g 2-Anthracen-9-yl-4-oxo-4*H*-benzo[*d*][1,3]oxazine-7-carboxylic acid [4-(1,3-oxazolin-2-yl)-phenyl]-amide. Die Substanz besitzt keinen Schmelzpunkt.
Die Struktur wurde mittels ¹H- und ¹³C-NMR-Spektroskopie überprüft.
Die Substanz reagiert mit äquimolaren Mengen Dodecylamin in Schmelze in einem Temperaturbereich von 100 - 220 °C unter Öffnung des Oxazinonrings und Erhalt der Oxazolingruppe.
Die Substanz reagiert mit äquimolaren Mengen Laurinsäure in Schmelze in einem Temperaturbereich von 150 - 280 °C unter Öffnung des Oxazolinrings und Erhalt der Oxazinongruppe.
Die Substanz ist als Fluoreszenzmarker geeignet.
2-Anthracen-9-yl-4-oxo-4*H*-benzo[*d*][1,3]oxazine-7-carboxylic acid [4-(1,3-oxazolin-2-yl)-phenyl]-amide zeigt in einer Dimethylformamidlösung im Fluoreszenzanregungsspektrum ein Maximum bei 385 nm und bei Anregung mit dieser Wellenlänge eine breite Emissionsbande mit einem Maximum bei 450 nm.
Das Umsetzungsprodukt von 2-Anthracen-9-yl-4-oxo-4*H*-benzo[*d*][1,3]oxazine-7-carboxylic acid [4-(1,3-oxazolin-2-yl)-phenyl]-amide mit Dodecylamin zeigt in einer Dimethylformamidlösung im Fluoreszenzanregungsspektrum ein Maximum bei 406 nm und bei Anregung mit dieser Wellenlänge eine breite Emissionsbande mit einem Maximum bei 450 nm.

## Patentansprüche

1. Multifunktionelle Kopplungsagenzien mit zyklischen Iminoester- und Iminoethergruppen der allgemeinen Struktur nach Formel 1, 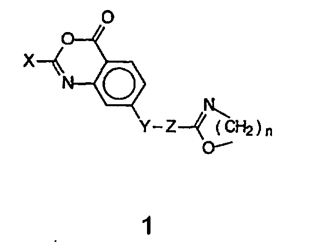 wobei X ein Aryl-, Aralkyl-, Aralkylen-, Alkyl- oder Alkylenrest mit 1 bis 42 Kohlenstoffatomen und 0 bis 12 Heteroatomen ist und eine oder mehrere zusätzliche, aktivierbare funktionelle Gruppen enthält, die für chemische Reaktionen oder physikalische Wechselwirkungen zur Verfügung stehen, Y eine -C(O)NH- oder -C(O)O-Gruppe oder eine chemische Bindung darstellt, Z ein Aryl-, Aralkyl- oder Alkylrest oder eine chemische Bindung und n gleich 2 oder 3 ist.

2. Multifunktionelle Kopplungsagenzien mit zyklischen Iminoester- und Iminoethergruppen nach Anspruch 1, bei denen Y-Z entsprechend der allgemeinen Struktur nach Formel 1 eine chemische Bindung darstellt, entsprechend der allgemeinen Struktur nach Formel 2

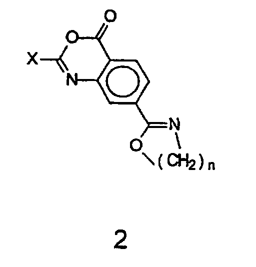

3. Multifunktionelle Kopplungsagenzien mit zyklischen Iminoester- und Iminoethergruppen nach Anspruch 1, bei denen Y entsprechend der allgemeinen Struktur nach Formel 1 eine -C(O)NH- Gruppe und Z entsprechend der allgemeinen Struktur nach Formel 1 ein 1.4-Phenylenrest und n gleich 2 ist, entsprechend der allgemeinen Struktur nach Formel 3

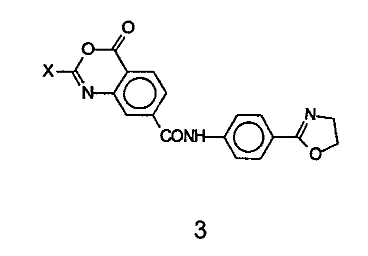

4. Multifunktionelle Kopplungsagenzien mit zyklischen Iminoester- und Iminoethergruppen nach einem der Ansprüche 1 - 3, bei denen die zusätzlichen, aktivierbaren funktionellen Gruppen von X entsprechend den allgemeinen Strukturen nach den Formeln 1, 2 oder 3 eine chemisch oder physikalisch vernetzbare Gruppe, eine polymerisierbare Gruppe, eine chromophore Gruppe, eine gegen Oxidation oder Radikalbildung stabilisierende Gruppe, eine mit spektroskopischen Methoden nachweisbare Gruppe, die als Marker dient, oder eine gegenüber Metallionen komplexierende Gruppe sind.

5. Verfahren zur Herstellung von multifunktionellen Kopplungsagenzien mit zyklischen Iminoester- und Iminoethergruppen nach einem der Ansprüche 1 bis 4, bei dem durch Umsetzung von Carbonsäurechloriden der allgemeinen Struktur nach Formel 4
X-COCl 4
mit Aminoterephthalsäure zu Verbindungen der allgemeinen Struktur nach Formel 5 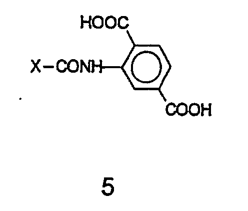 in einem inerten Lösungsmittel in Gegenwart eines Säurefängers und bei Temperaturen von 0 °C bis 150 °C und anschließlicher Umsetzung mit zugleich wasserentziehenden Chlorierungsagenzien bei Temperaturen von 0 °C bis 150 °C Zwischenprodukte der allgemeinen Struktur nach Formel 6 hergestellt werden, 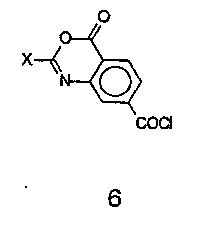 wobei X ein Aryl-, Aralkyl-, Aralkylen-, Alkyl- oder Alkylenrest mit 1 bis 42 Kohlenstoffatomen und 0 bis 12 Heteroatomen ist und eine oder mehrere zusätzliche, aktivierbare funktionelle Gruppen enthält, die für chemische Reaktionen oder physikalische Wechselwirkungen zur Verfügung stehen, und die hergestellten Zwischenprodukte mit 2-Ethanolamin oder 3-Propanolamin in einem inerten Lösungsmittel unter Zusatz eines Säurefängers bei Temperaturen 0 °C bis 150 °C, anschließender Reaktion mit wasserentziehenden Mitteln und nachträglicher Behandlung mit einer Base umgesetzt werden oder mit einem in 2-Stellung substituierten Oxazolin oder Oxazin, welches eine Hydroxylgruppe oder eine primäre Aminogruppe enthält, in einem inerten Lösungsmittel unter Zusatz eines Säurefängers bei Temperaturen von 0 °C bis 150 °C umgesetzt werden.

6. Verfahren nach Anspruch 5, bei dem als Säurefänger ein tertiäres Amin eingesetzt wird.

7. Verfahren nach Anspruch 5, bei dem zur Umsetzung der Verbindungen nach Formel 5 zu Verbindungen nach Formel 6 als wasserentziehendes Chlorierungsagens siedendes Thionylchlorid eingesetzt wird.

8. Verfahren nach Anspruch 5, bei dem nach Umsetzung der Verbindungen nach Formel 6 mit 2-Ethanolamin oder 3-Propanolamin als wasserentziehendes Mittel Thionylchlorid in Gegenwart eines inerten Lösungsmittels bei Temperaturen von 0 °C bis 20°C eingesetzt wird und zur nachträglichen Behandlung als Base eine wäßrige Bicarbonatlösung oder ein tertiäres Amin eingesetzt wird.

9. Verwendung von multifunktionellen Kopplungsagenzien mit zyklischen Iminoesterund Iminoethergruppen nach einem der Ansprüche 1 bis 4 als Kopplungsagenzien für Polymere, Oligomere und organische Verbindungen oder anorganische Stoffe mit Carboxyl-, Phenol-, Thiophenol- sowie Amino- und aliphatische Hydroxylgruppen

10. Verwendung nach Anspruch 9, bei dem die multifunktionellen Kopplungsagenzien mit zyklischen Iminoester- und Iminoethergruppen nach einem der Ansprüche 1 bis 4 für Kopplungsreaktionen in einem Temperaturbereich von 150°C bis 300°C in Polymerschmelzen oder -lösungen zur Kettenverlängerung, Verzweigung oder Vemetzung, zur Bildung von Blockcopolymeren, Multiblockcopolymeren oder Pfropfcopolymeren, insbesondere zur chemischen Kopplung von Polymeren mit unterschiedlichen funktionellen Gruppen, oder zur chemischen Anbindung von organischen und anorganischen Stoffen und darüber hinaus über die in den Kopplungsagenzien enthaltenen zusätzlichen, aktivierbaren funktionellen Gruppen, die für chemische Reaktionen oder physikalische Wechselwirkungen zur Verfügung stehen, für chemische Reaktionen oder physikalische Wechselwirkungen in den so hergestellten Polymeren, Polymerblends und Composites eingesetzt werden.

## Claims

1. Multifunctional coupling agents having cyclic iminoester and iminoether groups of the general structure according to formula 1, 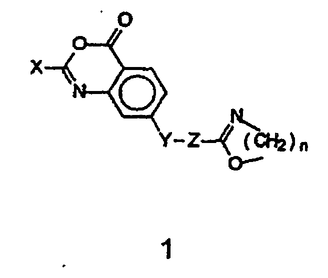 where X is an aryl, aralkyl, aralkylene, alkyl or alkylene radical having from 1 to 42 carbon atoms and from 0 to 12 heteroatoms and contains one or more additional, activatable functional groups which are available for chemical reactions or physical interactions, Y is a -C(O)NH or -C(O)O group or a chemical bond, Z is an aryl, aralkyl or alkyl radical or a chemical bond and n is 2 or 3.

2. Multifunctional coupling agents having cyclic iminoester and iminoether groups according to Claim 1, in which Y-Z in accordance with the general structure according to formula 1 is a chemical bond, in accordance with the general structure according to formula 2

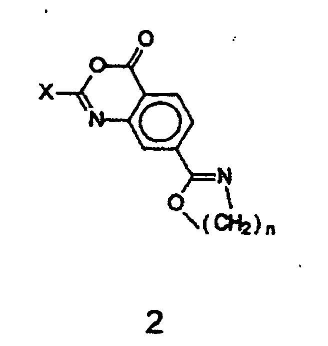

3. Multifunctional coupling agents having cyclic iminoester and iminoether groups according to Claim 1, in which Y in accordance with the general structure according to formula 1 is a -C(O)NH group and Z in accordance with the general structure according to formula 1 is a 1,4-phenylene radical and n is 2, in accordance with the general structure according to formula 3

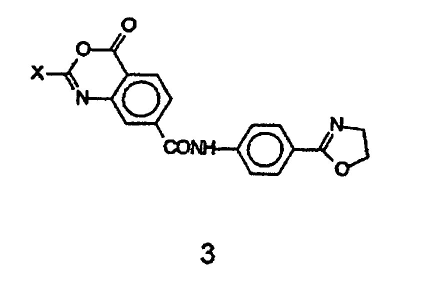

4. Multifunctional coupling agents having cyclic iminoester and iminoether groups according to any of Claims 1 to 3, in which the additional, activatable functional groups of X in accordance with the general structures according to formulae 1, 2 or 3 are a chemically or physically crosslinkable group, a polymerizable group, a chromophoric group, a group which provides stabilization with respect to oxidation or free-radical formation, a group detectable by spectroscopic methods and serving as a marker, or a group with complexing properties with respect to metal ions.

5. Process for preparing multifunctional coupling agents having cyclic iminoester and iminoether groups according to any of Claims 1 to 4, in which intermediates of the general structure according to formula 6 are prepared via reaction of carbonyl chlorides of the general structure according to formula 4
X-COCl 4
with aminoterephthalic acid to give compounds of the general structure according to formula 5 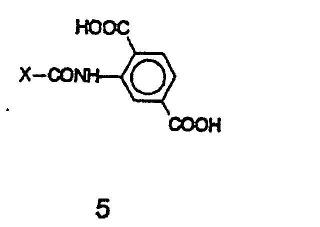 in an inert solvent in the presence of an acid scavenger and at temperatures of from 0°C to 150°C and subsequent reaction at temperatures of from 0°C to 150°C with chlorinating agents which simultaneously dehydrate, 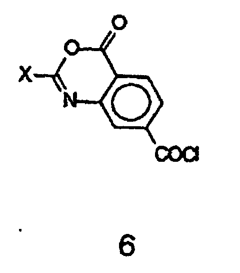 where X is an aryl, aralkyl, aralkylene, alkyl or alkylene radical having from 1 to 42 carbon atoms and from 0 to 12 heteroatoms and contains one or more additional, activatable functional groups which are available for chemical reactions or physical interactions, and the intermediates prepared are reacted with 2-ethanolamine or 3-propanolamine in an inert solvent with addition of an acid scavenger at temperatures of from 0°C to 150°C, and with subsequent reaction with dehydrating agents and post-treatment with a base, or are reacted with a 2-substituted oxazoline or oxazine which contains a hydroxy group or contains a primary amino group, in an inert solvent with addition of an acid scavenger at temperatures of from 0°C to 150°C.

6. Process according to Claim 5, in which a tertiary amine is used as acid scavenger.

7. Process according to Claim 5, in which boiling thionyl chloride is used as dehydrating chlorinating agent for the reaction of the compounds according to formula 5 to give compounds according to formula 6.

8. Process according to Claim 5, in which, after reaction of the compounds according to formula 6 with 2-ethanolamine or 3-propanolamine, as dehydrating agent, thionyl chloride is used in the presence of an inert solvent at temperatures of from 0°C to 20°C, and for the post-treatment, a tertiary amine or an aqueous bicarbonate solution is used as base.

9. Use of multifunctional coupling agents having cyclic iminoester and iminoether groups according to any of Claims 1 to 4 as coupling agents for polymers, oligomers and organic compounds or inorganic substances having carboxy, phenol, thiophenol, or else amino and aliphatic hydroxy groups.

10. Use according to Claim 9, in which the multifunctional coupling agents having cyclic iminoester and iminoether groups according to any of Claims 1 to 4 are used for coupling reactions in the temperature range from 150°C to 300°C in polymer melts or in polymer solutions, for chain extension, branching or crosslinking, for forming block copolymers, multiblock copolymers or graft copolymers, in particular for chemical coupling of polymers using various functional groups, or for chemical linkage of organic and inorganic substances and moreover, by way of the additional, activatable functional groups present in the coupling agents and available for chemical reactions or physical interactions, for chemical reactions or physical interactions in the resultant polymers, polymer blends and composites.

## Revendications

1. Agents de couplage multifonctionnels comportant des groupes cycliques iminoester et iminoéther de la structure générale répondant à la formule 1 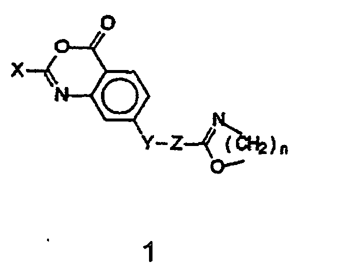 dans laquelle X représente un reste aryle, aralkyle, aralkylène, alkyle ou alkylène comportant de 1 à 42 atomes de carbone et de 0 à 12 hétéroatomes, et contenant un ou plusieurs autres groupes fonctionnels activables, lesquels sont disponibles pour des réactions chimiques ou des interactions physiques, Y représente un groupe - C(O)NH ou -C(O)O ou une liaison chimique, Z un reste aryle, aralkyle ou alkyle ou une liaison chimique, et n est égal à 2 ou 3.

2. Agents de couplage multifonctionnels comportant des groupes cycliques iminoester et iminoéther selon la revendication 1, dans lesquels Y-Z de la structure générale selon la formule 1 représente une liaison chimique, correspondant à la structure générale selon la formule 2

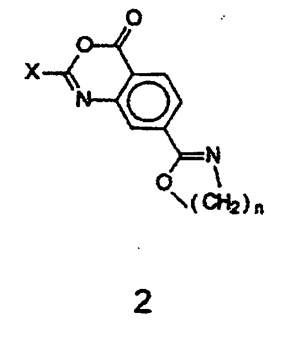

3. Agents de couplage multifonctionnels comportant des groupes cycliques iminoester et iminoéther selon la revendication 1, dans lesquels Y correspondant à la structure générale selon la formule 1 représente un groupe -C(O)NH et Z correspondant à la structure générale selon la formule 1 un reste 1,4-phénylène et n est égal à 2, correspondant à la structure générale selon la formule 3

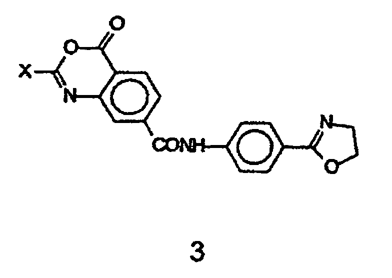

4. Agents de couplage multifonctionnels comportant des groupes cycliques iminoester et iminoéther selon l'une quelconque des revendications 1 à 3, dans lesquels les autres groupes fonctionnels activables de X correspondant aux structures générales selon les formules 1, 2 ou 3 représentent un groupe chimiquement ou physiquement réticulable, un groupe polymérisable, un groupe chromophore, un groupe stabilisant contre l'oxydation ou la formation de radicaux, un groupe qui peut être mis en évidence par des méthodes spectroscopiques, servant de marqueur, ou complexant vis-à-vis d'ions métalliques.

5. Procédé de préparation d'agents de couplage multifonctionnels comportant des groupes cycliques iminoester et iminoéther selon l'une quelconque des revendications 1 à 4, dans lequel on prépare, par réaction de chlorures d'acide carboxyliques de structure générale selon la formule 4
X-COCl 4
avec de l'acide aminotéréphtalique, pour former des composés de structure générale selon la formule 5 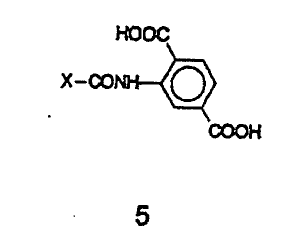 dans un solvant inerte, en présence d'un capteur d'acide et à des températures de 0°C à 150°C, et réaction subséquente avec des agents de chloration simultanément déshydratants, à des températures de 0°C à 150°C, des produits intermédiaires de structure générale selon la formule 6, 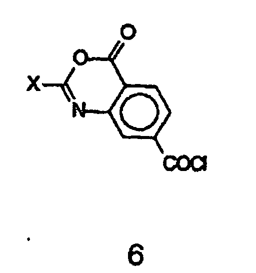 dans laquelle X représente un reste aryle, aralkyle, aralkylène, alkyle ou alkylène comportant de 1 à 42 atomes de carbone et de 0 à 12 hétéroatomes et contenant un ou plusieurs autres groupes fonctionnels activables, lesquels sont disponibles pour des réactions chimiques ou des interactions physiques, et les produits intermédiaires préparés sont mis à réagir avec de la 2-éthanolamine ou de la 3-propanolamine dans un solvant inerte avec addition d'un capteur d'acide à des températures de 0°C à 150°C, réaction subséquente avec des agents déshydratants et traitement subséquent par une base, ou sont mis à réagir avec une oxazoline ou une oxazine substituée en position 2, qui contient un groupe hydroxyle ou un groupe amino primaire, dans un solvant inerte avec addition d'un capteur d'acide à des températures de 0°C à 150°C.

6. Procédé selon la revendication 5, dans lequel on utilise comme capteur d'acide une amine tertiaire.

7. Procédé selon la revendication 5, dans lequel, pour la réaction des composés selon la formule 5 pour former des composés selon la formule 6, on met en oeuvre comme agent de chloration déshydratant du chlorure de thionyle bouillant.

8. Procédé selon la revendication 5 dans lequel, après réaction des composés selon la formule 6 avec de la 2-éthanolamine ou de la 3-propanolamine, on met en oeuvre comme moyen déshydratant du chlorure de thionyle en présence d'un solvant inerte à des températures de 0°C à 20°C et, pour le traitement ultérieur, on met en oeuvre en tant que base une solution aqueuse de bicarbonate ou une amine tertiaire.

9. Utilisation d'agents de couplage multifonctionnels comportant des groupes cycliques iminoester et iminoéther selon l'une quelconque des revendications 1 à 4 comme agents de couplage pour polymères, oligomères et composés organiques ou substances inorganiques avec des groupes carboxyle, phénol, thiophénol ainsi qu'amino et hydroxyle aliphatiques.

10. Utilisation selon la revendication 9, dans laquelle les agents de couplage multifonctionnels comportant des groupes cycliques iminoester et iminoéther selon l'une quelconque des revendications 1 à 4 sont mis en oeuvre pour des réactions de couplage dans une plage de température de 150°C à 300°C dans des masses fondues ou des solutions de polymères à des fins d'allongement de chaîne, de ramification ou de réticulation, pour la formation de copolymères blocs, de copolymères multiblocs ou de copolymères greffés, en particulier pour le couplage chimique de polymères comportant différents groupes fonctionnels, ou pour l'attachement chimique de composés organiques et inorganiques et, en outre, via d'autres groupes fonctionnels activables contenus dans les agents de couplage, et qui sont disponibles pour des réactions chimiques ou des interactions physiques, pour des réactions chimiques ou des interactions physiques dans les polymères, les mélanges de polymères et les composites ainsi préparés.
